Europäisches Patentamt

(19) European Patent Office

Office européen des brevets

(11) Publication number: **0 003 150**
**B1**

# (12) EUROPEAN PATENT SPECIFICATION

(45) Date of publication of patent specification: **25.11.81**

(51) Int. Cl.³: **A 61 K 31/73, A 61 K 9/00**

(21) Application number: **79200014.3**

(22) Date of filing: **10.01.79**

(54) Stabilized aqueous parenteral antibiotic compositions and a process for their preparation.

(30) Priority: **16.01.78 US 869741**

(43) Date of publication of application:
**25.07.79 Bulletin 79/15**

(45) Publication of the grant of the European patent:
**25.11.81 Bulletin 81/47**

(84) Designated Contracting States:
**BE CH DE FR GB IT LU NL SE**

(56) References cited:
**DE - A - 2 437 144**
**FR - A - 2 120 132**
**FR - A - 2 146 373**

(73) Proprietor: **SCHERICO LTD.**
**Töpferstrasse 5**
**CH-6004 Lucerne (CH)**

(72) Inventor: **Rosenkrantz, Bernard Eli**
**31 Hyde Road**
**Bloomfield New Jersey 07003 (US)**
Inventor: **Stupak, Elliot Isaac**
**1 Essex Place**
**West Caldwell New Jersey 07006 (US)**

(74) Representative: **Antony, Fritz, Dr. et al,**
**P.O. Box 601 Winkelriedstrasse 35**
**CH-6002 Lucerne (CH)**

Courier Press, Leamington Spa, England.

# O 003 150

Stabilized aqueous parenteral antibiotic compositions and a process for their preparation

This invention relates to color-stabilized aqueous parenteral antibiotic compositions of a parenterally acceptable salt of an aminoglycoside antibacterial agent, having a pyranose ring which is unsaturated between the 4' and 5' positions and which contains an aminoalkyl substituent at the 5' position and to a process for their preparation.

Aminoglycosides have long been recognized as effective antibacterial agents. Interest within this series has recently focused on a more potent subclass which is characterized by the presence of a pyranose ring which is unsaturated between the 4' and 5' positions and wherein the 5' position bears an amino alkyl substituent. Example of known antibiotics of this class are siosmicin (U.S. Patent No. 3,832,286); verdamicin (British Patent No, 1,405,283); Antibiotic G—52 (U.S. Patent No. 3,956,068); Antibiotic 66—40B and Antibiotic 66—40D (U.S. Patent No. 3,880,828); and Mu-1, Mu-2, Mu-4, Mu-5 and Mu-6 (5-epi-sisomicin), formerly known as Mutamicins (South African Patent No. 74/4938). For purpose of this specification, the term "antibiotic" will also include synthetic derivatives such as the 1-N-alkyl and 1-N-acyl derivatives, e.g. 1-N-ethyl-sisomicin (netilmicin); (South African Patent No. 74/4939). These antibiotics are generally administered in the form of a parenterally acceptable acid addition salt, e.g. sisomicin sulfate and netilmicin sulfate.

Unfortunately, these unsaturated aminoglycosides are prone to decomposition, particularly when exposed to the air for even relatively short periods of time and particularly at elevated temperatures. This decomposition results in the rapid formation of highly colored impurities (chromophonic material), eventually resulting in a loss of potency and pharmaceutical elegance, along with the production of materials of unknown toxicology. At the same time the pH of the mixture tends to fall below acceptable levels. Accordingly, the aminoglycoside compositions of this subclass which have been marketed, namely such comprising sisomicin sulfate, had a comparatively short shelf life. It would be highly desirable to significantly increase the shelf life of such antibiotic systems. For purpose of this specification, "shelf life" refers to the length of time in which the color of the formulation is below 1200 APHA Color Unites and the pH remains above 2.5. (The APHA Color Unit is a widely recognized standard described for example in "Standard method for the examination of water and waste-water", 13th Edition, 1971, American Public Health Association.)

We have now surprisingly found that highly pH and color-stable parenteral aqueous solutions of the subject unsaturated aminoglycosides are obtained if the initial pH of the system is brought between 5.0 and 7.0. A more preferred pH range is between 5.8 and 6.8, and, most preferred, the pH is between 6.2 and 6.5. The resultant shelf life significantly exceeds that of the aforementioned prior art formulation having an initial pH of less than 5.0, or a formulation having a pH greater than 7.0. In the preferred pH range between 5.8 and 6.8, the pH is found to be particularly stable and the product thereby has more predictable characteristics. In the most preferred pH range between 6.2 and 6.5, the product is found to have a particularly stable pH even in the presence of oxygen, e.g. air.

Aminoglycosides have classically been marketed with initial pH's in the range of about 3.5 to 4.5, although formulations with a higher pH have been described in the more recent art.

The unexamined German Patent Application (DOS) No. 2.437.144 deals with the problem of reducing renal side effects of aminoglycosides and proposes, as a solution, that glucosaccharinic acid is applied together with the antibiotic. The acid may be administered separately or in admixture with the antibiotic whereby the glucosaccharinic acid when used alone or in the combination should preferably have a pH of 6.5 to 7.5. Equally the unexamined French Patent Application No. 2.120.132 indicates that the slight coloring which may occur upon long storage in formulations containing saturated aminoglycosides may be counteracted by adding one or more polyols to the mixture whereby, for general reasons (e.g. pharmaceutical), the pH should be close to neutral.

Nowhere in the prior art, however, was it indicated that both colour-and pH-stability of unsaturated aminoglycoside formulations can be greatly improved by the teaching of the present invention, i.e. by brining the initial pH of the solution to a value between 5.0 and 7.0.

Particularly stable compositions are obtained if also antioxidants are added to the formulation, particularly at higher concentrations of the antibiotic. The preferred antioxidants are sodium metabisulfite, sodium bisulfite and sodium sulfite, or combinations thereof, with the choice of salt largely depending upon the initial pH of the system which is to be stabilized. These antioxidant agents act by being either preferentially oxidized (reducing agents), and thereby gradually used up, or by blocking an oxidative chain reaction. Other suitable antioxidants for an aqueous system are sodium thiosulfate, sodium formaldehyde sulfoxylate, acetone sodium metabisulfite, ascorbic acid, isoascorbic acid, thioglycerol, thiosorbitol, thioglycolic acid and cysteine hydrochloride. This finding is particularly surprising since the prior art (FR. 2.120.132) emphasizes that in formulations containing saturated aminoglycoside the addition of sulfite salts leads to highly pH-unstable solutions. The desired pH range can be achieved by upward adjustment of the pH with a suitable base such as sodium hydroxide or downward adjustment with a suitable acid such as hydrochloric acid. In a preferred embodiment, the desired initial pH is established by appropriately selecting an antioxidant or combination of antioxidants which will give the desired pH without need for a discrete pH adjustment.

Three preferred aminoglycoside compositions of this invention involve sisomicin sulfate in a

2

concentration of 10—50 mg/ml, netilmicin sulfate in a concentration of 10—100 mg/ml and 5-epi-sisomicin in a concentration of 10—50 mg/ml.

Numerous other parenterally acceptable ingredients in usual amounts can be optionally added to the composition, such as preservatives, e.g. parabens, benzyl alcohol; electrolytes to make the solutions isotonic with body fluids, e.g. sodium chloride and sodium sulfate; and chelating agents, e.g. disodium EDTA.

The particular stability in the preferred pH range between 6.2 and 6.5 is illustrated by the following experiment:

Two solutions containing 250 mg netilmicin sulfate in 5 ml of water were prepared and the pH was adjusted to 6.5 by adding NaOH. One solution was filled into an ampoule and sealed under nitrogen, whereas the other was sealed in the presence of air. The ampoules were stored for 1 week at 76°C after which time the pHs of the solutions were:
a) of the one sealed under nitrogen: still 6.5;
b) of the one sealed in the presence of air: 6.2.

The formulation of the aforesaid commercially available aqueous solutions of sisomicin sulfate is shown below. These solutions have an initial pH of 3.7—3.9. The compositions have a label-recommended shelf life of 18 months at 30°C which is much less than the shelf life of the improved system, e.g. Example 1.

PRIOR ART

| Injectable Solution | Per 1.0 ml |
|---|---|
| Sisomicin (charged as sisomicin sulfate) | 50.0 mg |
| Sodium Metabisulfite | 3.0 mg |
| Sodium Chloride | 3.6 mg |
| Methylparaben | 0.8 mg |
| Propylparaben | 0.1 mg |
| Disodium Edetate | 0.1 mg |
| Distilled Water, q.s. add | 1.0 ml |

Example 1

A composition is prepared by adjusting the pH of the above prior art formulation to 5.2 by the addition of a 0.1N sodium hydroxide solution. The resultant formulation has a shelf life of at least 36 months at 30°C.

Example 2

A pharmaceutical composition is prepared by blending together the following ingredients in the manner hereinafter indicated.

| Injectable Solution | Per 1.0 mls | Per 50 litres |
|---|---|---|
| Sisomicin (charged as sisomicin sulfate) | 50.0 mg | 2.500 g |
| Sodium sulfite | 0.8 mg | 40 g |
| Sodium metabisulfite | 2.4 mg | 120 g |
| Propyl paraben | 0.1 mg | 5 g |
| Methyl paraben | 0.8 mg | 40 g |
| Disodium EDTA | 0.1 mg | 5 g |
| Sodium chloride | 3.9 mg | 195 g |
| Distilled water, q.s. | 1.0 ml | 50 liters |

3

*Procedure:* For a 50 liter batch

Charge approximately 35 liters of the distilled water to a suitable stainless steel jacketed vessel and heat to about 70°C. Charge the methylparaben and propylparaben to the heated water for injection and dissolve with agitation. When the parabens are completely dissolved, cool the contents of the tank to 25°C—30°C. Sparge the solution with nitrogen gas and keep covered with nitrogen during subsequent processing. Charge and dissolve the disodium EDTA, sodium chloride, sodium sulfite and sodium metabisulfite. Charge and dissolve the sisomicin sulfate. Bring the batch volume up to 50 liters with the distilled water and agitate until homogenous. Under sterile conditions, filter the solution through a suitable bacteria retentive filter collecting the filtrate in a filling tank.

Fill the product aseptically into sterile pyrogen free multiple dose vials, ampoules or syringes and seal.

A composition prepared according to Example 2 has an initial pH of about 5.2 and a shelf life of at least 36 months at 30°C. This procedure yields a formulation in the desired pH range without need for a discrete pH adjustment step.

The formulations 3 to 21 given below were prepared according to the procedure of Example 2. All had a shelf life of at least 36 months at 30°C.

TABLE I

| | mg Antibiotic | Sodium sulfite mg/ml | Sodium metabisulfite mg/ml | Sodium chloride mg/ml | Sodium sulfate mg/ml | Methyl-paraben mg/ml | Propyl-paraben mg/ml | Disodium EDTA mg/ml | Initial pH |
|---|---|---|---|---|---|---|---|---|---|
| 3 | Sisomicin sulfate 50 | 3.9 | – | – | 6.4 | 0.8 | 0.1 | 0.1 | 6.1 |
| 4 | Sisomicin sulfate 10 | 0.8 | 2.4 | 5.8 | – | 1.3 | 0.2 | 0.1 | 5.5 |
| 5 | Netilmicin sulfate 10 | 4.0 | – | 5.4 | – | 1.3 | 0.2 | – | 6.8 |
| 6 | Netilmicin sulfate 10 | 0.8 | 2.4 | 6.1 | – | – | – | – | 5.5 |
| 7 | Netilmicin sulfate 10 | 1.2 | 2.1 | 6.1 | – | – | – | – | 5.8 |
| 8 | Netilmicin sulfate 10 | 1.2 | 2.1 | – | 19.4 | – | – | – | 6.0 |
| 9 | Netilmicin sulfate 10 | 0.8 | 2.4 | – | 19.4 | – | – | – | 5.8 |
| 10 | Netilmicin sulfate 10 | 2.8 | 0.9 | 5.9 | – | – | – | – | 6.3 |
| 11 | Netilmicin sulfate 25 | 0.8 | 2.4 | – | 2.6 | 1.3 | 0.2 | 0.1 | 5.4 |
| 12 | Netilmicin sulfate 25 | 1.2 | 2.1 | – | 2.6 | 1.3 | 0.2 | 0.1 | 5.7 |
| 13 | Netilmicin sulfate 25 | 1.2 | 2.1 | 1.5 | – | 1.3 | 0.2 | 0.1 | 5.6 |
| 14 | Netilmicin sulfate 25 | 2.0 | – | 3.8 | – | – | – | – | 6.3* |
| 15 | Netilmicin sulfate 25 | 4.0 | – | 4.2 | – | 1.3 | 0.2 | – | 6.4 |
| 16 | Netilmicin sulfate 25 | 3.2 | 0.6 | 4.2 | – | 1.3 | 0.2 | – | 6.3 |
| 17 | Netilmicin sulfate 50 | 4.0 | – | 2.5 | – | 1.3 | 0.2 | – | 6.2 |
| 18 | Netilmicin sulfate 50 | 4.0 | – | 2.2 | – | 1.3 | 0.2 | – | 6.3 |
| 19 | Netilmicin sulfate 100 | 2.0 | – | – | – | – | – | – | 6.3* |
| 20 | Netilmicin sulfate 100 | 4.0 | – | – | – | 1.3 | 0.2 | – | 6.0 |
| 21 | Netilmicin sulfate 100 | 4.0 | – | – | – | 0.8 | 0.1 | – | 6.3 |

* These formulations also include 10 mg benzylalcohol /ml. Whenever necessary for accurate adjustment of the pH, NaOH or HCl was used.

## Claims

1. A pH- and color-stabilized parenteral aqueous antibiotic composition of a parenterally acceptable salt of an aminoglycoside antibacterial agent having a pyranose ring which is unsaturated between the 4' and 5' positions and which contains an amino alkyl substituent at the 5' position, characterized in that the initial pH of the composition is between 5.0 and 7.0, with the proviso that the composition does not contain any glucosaccharinic acid.

2. A composition according to claim 1, characterized in that an antioxidant is included.

3. A composition according to claim 2, characterized in that a sulfite salt or a mixture of sulfite salts is used as antioxidant.

4. A composition according to claim 3, characterized in that said composition comprises 2.4 mg/ml of solution of sodium metabisulfite and 0.8 mg/ml of solution of sodium sulfite.

5. A composition according to any one of claims 1 to 4, characterized in that the pH is in the range of 5.8—6.8.

6. A composition according to any one of claims 1 to 5, characterized in that the pH is in the range of 6.2—6.5.

7. A composition according to any one of claims 1 to 6, characterized in that the aminoglycoside antibacterial agent is a salt of sisomicin or of 5-episisomicin.

8. A composition according to claim 7, characterized in that said salt is sisomicin sulfate in a concentration of 10 to 50 mg/ml of solution.

9. A composition according to any one of claims 1 to 6, characterized in that the aminoglycoside antibacterial agent is a salt of netilmicin.

10. A composition according to claim 9, characterized in that said salt is netilmicin sulfate in a concentration of 10—100 mg/ml of solution.

11. Process for the preparation of a color- and pH-stabilized parenteral aqueous antibiotic composition of a parenterally acceptable salt of an aminoglycoside antibacterial agent having a pyranose ring which is unsaturated between the 4' and 5' positions and which contains an aminoalkyl substituent at the 5' position, characterized in that the pH of the composition is adjusted to give an initial pH between 5.0 and 7.0, with the proviso that the composition does not contain any glucosaccharinic acid.

## Revendications

1. Composition antibiotique aqueuse parentérale à pH et couleur stabilisés d'un sel acceptable par voie parentérale d'un agent antibactérien d'aminoglycoside ayant un noyau pyranose qui est insaturé entre les positions 4' et 5' et qui contient un substituant aminoalcoyle à la position 5', caractérisée en ce que le pH initial de la composition est compris entre 5,0 et 7,0, à condition que la composition ne contienne aucun acid glucosaccharinique.

2. Composition selon la revendication 1, caractérisée en ce qu'un agent antioxydant est incorporé.

3. Composition selon la revendication 2, caractérisée en ce qu'un sel de sulfite ou un mélange de sels de sulfite est utilisé comme agent antioxydant.

4. Composition selon la revendication 3, caractérisée en ce qu'elle contient 2,4 mg/ml d'une solution de métabisulfite de sodium et 0,8 mg/ml de solution de sulfite de sodium.

5. Composition selon l'une quelconque des revendications 1 à 4, caractérisée en ce que le pH est compris entre 5,8 et 6,8.

6. Composition selon l'une quelconque des revendications 1 à 5, caractérisée en ce que le pH est comprise entre 6,2 et 6,5.

7. Composition selon l'une quelconque des revendications 1 à 6, caractérisée en ce que l'agent antibactérien d'aminoglycoside est un sel de sisomicine ou de 5-épi-sisomicine.

8. Composition selon la revendication 7, caractérisée en ce que ledit sel est du sulfate de sisomicine à une concentration de 10 à 50 mg/ml de la solution.

9. Composition selon l'une quelconque des revendications 1 à 6, caractérisée en ce que l'agent antibactérien d'aminoglycoside est un sel de nétilmicine.

10. Composition selon la revendication 9, caractérisée en ce que le sel précité est du sulfate de nétilmicine à une concentration de 10 à 100 mg/ml de la solution.

11. Procédé pour la préparation d'une composition antibiotique aqueuse parentérale stabilisée en couleur et en pH, d'un sel acceptable par voie parentérale d'un agent antibactérien d'aminoglycoside ayant un noyau pyranose qui est insaturé entre les positions 4' et 5' et qui contient un substituant d'aminoalcoyle à la position 5', caractérisé en ce que le pH de la composition est ajusté pour donner un pH initial entre 5,0 et 7,0, à condition que la composition ne contienne aucun acide glucosaccharinique.

## Patentansprüche

1. Eine pH- und farbstabilisierte, parenterale wäßrige antibiotische Zubereitung eines parenteral verträglichen Salzes eines antibakteriellen Aminoglykosid-Wirkstoffs mit einem Pyranose-Ring, der

6

zwischen den 4'- und 5'-Stellungen ungesättigt ist und einen Aminoalkyl-Substituenten in der 5'-Stellung trägt, dadurch gekennzeichnet, daß der anfängliche pH der Zubereitung zwischen 5,0 und 7,0 liegt, mit der Maßgabe, daß die Zubereitung keinerlei Gluco-saccharinsäure enthält.

2. Zubereitung nach Anspruch 1, dadurch gekennzeichnet, daß sie zusätzlich ein Antioxidationsmittel enthält.

3. Zubereitung nach Anspruch 2, dadurch gekennzeichnet, daß als Antioxidationsmittel ein Sulfit-Salz oder eine Mischung von Sulfit-Salzen verwendet wird.

4. Zubereitung nach Anspruch 3, dadurch gekennzeichnet, daß sie 2,4 mg/ml einer Lösung von Dinatriumdisulfit (sodium metabisulfite) und 0,8 mg/ml einer Lösung von Natriumsulfit enthält.

5. Zubereitung nach Anspruch 1 bis 4, dadurch gekennzeichnet, daß der pH im Bereich von 5,8 bis 6,8 liegt.

6. Zubereitung nach Anspruch 1 bis 5, dadurch gekennzeichnet, daß der pH in Bereich von 6,2 bis 6,5 liegt.

7. Zubereitung nach Anspruch 1 bis 6, dadurch gekennzeichnet, daß der antibakterielle Aminoglykosid-Wirkstoff ein Salz des Sisomicins oder des 5-Epi-sisomicins ist.

8. Zubereitung nach Anspruch 7, dadurch gekennzeichnet, daß das Salz Sisomicin-sulfat in einer Lösung der Konzentration 10 bis 50 mg/ml ist.

9. Zubereitung nach Anspruch 1 bis 6, dadurch gekennzeichnet, daß der antibakterielle Aminoglykosid-Wirkstoff ein Salz des Netilmicins ist.

10. Zubereitung nach Anspruch 9, dadurch gekennzeichnet, daß das Salz Netilmicin-sulfat in einer Lösung der Konzentration 10 bis 100 mg/ml ist.

11. Verfahren zur Herstellung einer farb- und pH-stabilisierten, parenteralen wäßrigen antibiotischen Zubereitung eines parenteral verträglichen Salzes eines antibakteriellen Aminoglykosid-Wirkstoffes mit einem Pyranose-Ring, der zwischen den 4'- und 5'-Stellungen ungesättigt ist und in 5'-Stellung einen Aminoalkyl-Substituenten trägt, dadurch gekennzeichnet, daß der pH der Zubereitung auf einen anfänglichen pH zwischen 5,0 und 7,0 eingestellt wird, mit der Maßgabe, daß die Zubereitung keinerlei Gluco-saccharinsäure enthält.